# EUROPÄISCHE PATENTSCHRIFT

(11) **EP 0 862 908 B1**
(45) Veröffentlichungstag und Bekanntmachung des Hinweises auf die Patenterteilung: **09.06.2004**
(21) Anmeldenummer: 98103628.8
(22) Anmeldetag: 02.03.1998
(51) Int. Cl.: A61K 7/06

(54) **Polysiloxanhaltige Haarfestigungsmittel**
Hair fixative with a polysiloxane
Fixateur capillaire contenant un polysiloxane

(30) Priorität: 07.03.1997 DE 19709277
(43) Veröffentlichungstag der Anmeldung: 09.09.1998
(73) Patentinhaber: BASF AKTIENGESELLSCHAFT, 67056 Ludwigshafen (DE)
(72) Erfinder: Sanner, Axel, Dr., 67227 Frankenthal (DE); Müller, Wolfgang, Dr., 67227 Frankenthal (DE); Schehlmann, Volker, Dr., 67354 Römerberg (DE)

(56) Entgegenhaltungen:
- EP-A- 0 524 612
- EP-A- 0 531 763
- WO-A-92/16187
- US-A- 5 578 298
- US-A- 5 662 892
- US-A- 5 851 516
- DATABASE CHEMICAL ABSTRACTS [Online] stn abstract 112: 104 578, XP002133154 & JP 01 190619 A (SHISEIDO CO., LTD) 31. Juli 1989 (1989-07-31)

## Beschreibung

Für die Festigung von Haarfrisuren werden seit fast 50 Jahren erfolgreich synthetische Polymere eingesetzt. Während in der Anfangszeit bevorzugt Vinyllactam-Homo- und Copolymere zum Einsatz gelangten, haben später carboxylatgruppenhaltige Polymere zunehmend an Bedeutung gewonnen. Das gewünschte Eigenschaftsprofil wie starke Festigung bei hoher Luftfeuchtigkeit, Elastizität, Auswaschbarkeit vom Haar und Verträglichkeit mit den übrigen Formulierungskomponenten werden durch Copolymerisation einer Kombination von hydrophoben, elastifizierenden und carboxylgruppen enthaltenden Monomeren erzielt (Lit: Robert Y. Lochhead, Vol. 103, December 1988, 24 ff., Rolf-Dieter Reinhardt Cosmetics and Toiletries Manufacture World Wide 1995, 189m ff.).

Während die obengenannten Anforderungen heute von verschiedenen Polymertypen erreicht werden, wird immer häufiger der Griff der mit diesen Polymeren gefestigten Frisuren als unangenehm stumpf und "unnatürlich" empfunden. Versuche durch Zusätze zu den Formulierungen zu einer Verbesserung zu gelangen, führten bisher nicht zu voll befriedigenden Ergebnissen: Die Zugabe üblicher Weichmacher verbessert zwar den Griff, reduziert aber gleichzeitig in vielen Fällen die Festigungswirkung. Die häufig eingesetzten Polysiloxane sind mit den polaren Polymeren nicht verträglich und verlangen oft weitere Zusätze um überhaupt formuliert werden zu können. Entmischungen können sowohl während der Lagerung der Formulierung als auch während des Gebrauchs zu Problemen führen (vgl. EPA 0408311, S.1, Zeile 25 ff).

Es hat daher nicht an Versuchen gefehlt, Polysiloxangruppen kovalent an das Festigerpolymer zu binden, um Entmischungen zu verhindern. Z. B. beschreibt die europäische Patentanmeldung 0408311 Haarpflege-Polymere aus einem Polysiloxangruppen enthaltendem Monomer mit den üblichen hydrophilen und hydrophoben Monomeren. In den Europäischen Patentanmeldungen EP 0412704 bis EP 0412707 wird vorgeschlagen, Polysiloxangruppen in Form von Makromonomeren mit Molmassen von 1000 bis 50000 ebenfalls mit hydrophoben und hydrophilen Monomeren zu polymerisieren.

Die Synthese der Siloxangruppen enthaltenden Makromonomeren ist außerordentlich aufwendig. Aus den Polymeren können nicht umgesetzte Makromonomere und deren unreaktive Verunreinigungen aufgrund ihres hohen Molekulargewichts kaum abgetrennt werden. Sie stellen ein toxikologisches und allergenes Risiko dar und verschlechtern die Löslichkeit. Darüber hinaus sind die erhaltenen Copolymeren, um eine gute Wirkung zu erzielen, oft nur in Kombination mit weiteren Polymeren, Carriern und weiteren Hilfsmittel zu formulieren, wie die o.g. Patentschriften lehren.
Aufgabe der Erfindung sind polysiloxanhaltige Haarfestiger-Formulierungen ohne die geschilderten Nachteile.

Die Patentschrifte WO-A-9216187, EP-A-0524612 und JP-A-01190619 beschreiben haarkosmetische Zusammensetzungen aus einem Copolymer und einem Aminogruppenhaltigen Polysiloxan.

Die Erfindung betrifft: Haarfestigungsmittel enthaltend neben üblichen Hilfs- und Zusatzstoffen
(A) 0,5 bis 15 Gew.-% carboxylgruppenhaltige Polymere, die in neutralisierter Form wasserlöslich oder wasserdispergierbar sind, mit einer Glasübergangstemperatur über 0° C,
(B) 0,5 bis 30 Gew.-% bezogen auf das eingesetzte Polymer (A) primäre, sekundäre oder tertiäre Aminogruppen enthaltende Polysiloxane, der folgenden Struktur 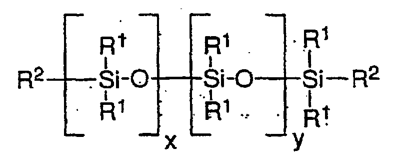 wobei die Reste R¹ identisch oder unterschiedlich sein können und aus der Gruppe Methyl, Ethyl, Propyl, Butyl ausgewählt sind, R² Gruppen der allgemeinen Formel -(CH₂)ₙ-N(R⁴)₂ mit R⁴ gleich oder verschieden in der Bedeutung H oder aliphatische Kohlenwasserstoffreste mit 1-20 Kohlenstoffatomen,
   n = 0-10 bedeuten und
   x,y so gewählt sind, das das Molekulargewicht des Polysiloxan-Blocks (B) zwischen 300 und 30000 liegt;
(C) 0 bis 50 % eines niedermolekularen Neutralisierungsmittels.

Geeignete carboxylatgruppenhaltige Polymere (A) sind beispielsweise Copolymere von Crotonsäure mit Vinylestern, (Meth)acrylsäure/(Meth)acrylester gegebenenfalls mit weiteren stickstoffhaltigen Comonomeren und Halbester von Copolymeren von Maleinsäure mit Vinylethern, wie sie üblicherweise in Haarfestiger Sprays Schäumen und Gelen eingesetzt werden (siehe.: Robert Y. Lochhead, Vol. 103, December 1988, 24 ff., Rolf-Dieter Reinhardt Cosmetics and Toiletries Manufacture World Wide 1995, 189m ff.).

Weiterhin geeignet sind Carboxylgruppen enthaltende Polyurethane, wie sie in der deutschen Patentschrift DE 4225045 beschrieben sind Es können aber auch speziell auf den erfindungsgemäßen Einsatz als Haarfestigungsmittel abgestimmte Polymere eingesetzt werden. Dabei kann etwa durch Erhöhung des Carboxylatanteils die Auswaschbarkeit verbessert oder durch Modifizierung des hydrophoben Monomeranteils die Festigung beeinflußt werden.

Wichtig ist daß die Polymeren in neutralisierter Form eine Glasübergangstemperatur von über 0° C aufweisen, bevorzugt größer 10°C und insbesondere größer 20°C.

Die erfindungsgemäßen Haarfestigungsmittel enthalten die Polymere (A) in einer Menge von 0,5-15, bevorzugt von 2-10 Gew.-%.

Geeignete aminogruppenhaltige Siloxane (B) sind insbesondere solche der folgenden Struktur 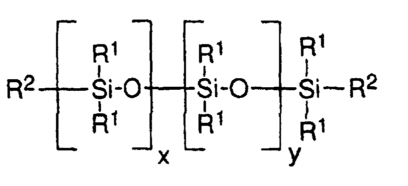 wobei die Reste R¹ identisch oder unterschiedlich sein können, und aus der Gruppe Methyl, Ethyl, Propyl, Butyl, ausgewähltsind.

Die Reste R² sind Gruppen der allgemeinen Formel -(CH₂)ₙ -N(R⁴)₂, wobei R4 gleich -H, oder aus der Gruppe der aliphatischen Kohlenwasserstoffe mit 1 bis 20 Kohlenstoffatomen, aromatischer Natur oder eine Aminoalkyl-Gruppe mit 1-12 Kohlenstoffatomen sein kann, und n eine ganze Zahl zwischen 0 und 10 ist.

Die Reste R⁴ können gleich oder verschieden sein.

x und y sind ganze Zahlen derart, daß das Molekulargewicht des Polysiloxan-Blocks zwischen 300 und 30000 liegt.

Besonders geeignete Siloxane sind solche, der allgemeinen Struktur: 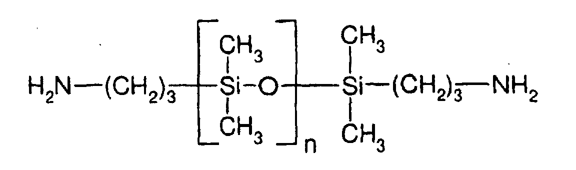 Geeignete Neutralisationsmittel (C) sind zum einen Mineralbasen wie Natriumcarbonat oder Kaliumcarbonat sowie Ammoniak, zum anderen organische Basen wie zum Beispiel Aminoalkohole speziell 2-Amino-2-methyl-1-propanol (AMP), Triethanolamin, Triisopropanolamin (TIPA), Monoethanolamin, Diethanolamin, Tri(2-hydroxy-1-propyl)amin, 2-Amino-2-methyl-1,3-propandiol (AMPD) oder 2-Amino-2-hydroxymethyl-1,3-propandiol sowie Diamine wie zum Beispiel Lysin.

Für die Verwendung in den erfindungsgemäßen Haarfestigungsmitteln werden die Carboxylgruppen der Copolymerisate, zweckmäßigerweise zu 5 bis 100 %, vorzugsweise zu 30 bis 95 %, neutralisiert.

Die erfindungsgemäßen Mischungen kommen beispielsweise als Haargele, flüssige Haarfestiger, Haarschäume und vor allem als Frisurenfestiger in Form von Sprayzubereitungen zur Anwendung.

In vielen Fällen ist es zweckmäßig, zusätzlich weitere Polymere in Anteilen von 0,1 bis 15 Gew.-%, vorzugsweise 1 bis 5 Gew.-% bezogen auf die Formulierung, einzusetzen.

Geeignete Zusatzpolymere sind vor allem Polymere und Copolymere von N-Vinyllactamen und Vinyl-Amiden wie z.B Vinylpyrrolidon, Vinylcaprolactam, Vinylformamid und Vinyl(meth)acetamid. Weiterhin kommen als zusätzliche Polymere sulfogruppenhaltige Polyamide, Polyurethane und Polyester, wie sie z. B. in den Patentschriften EP 0696607, bzw., DE 4225045 bzw. US 3 779 993 beschrieben sind, in Betracht.

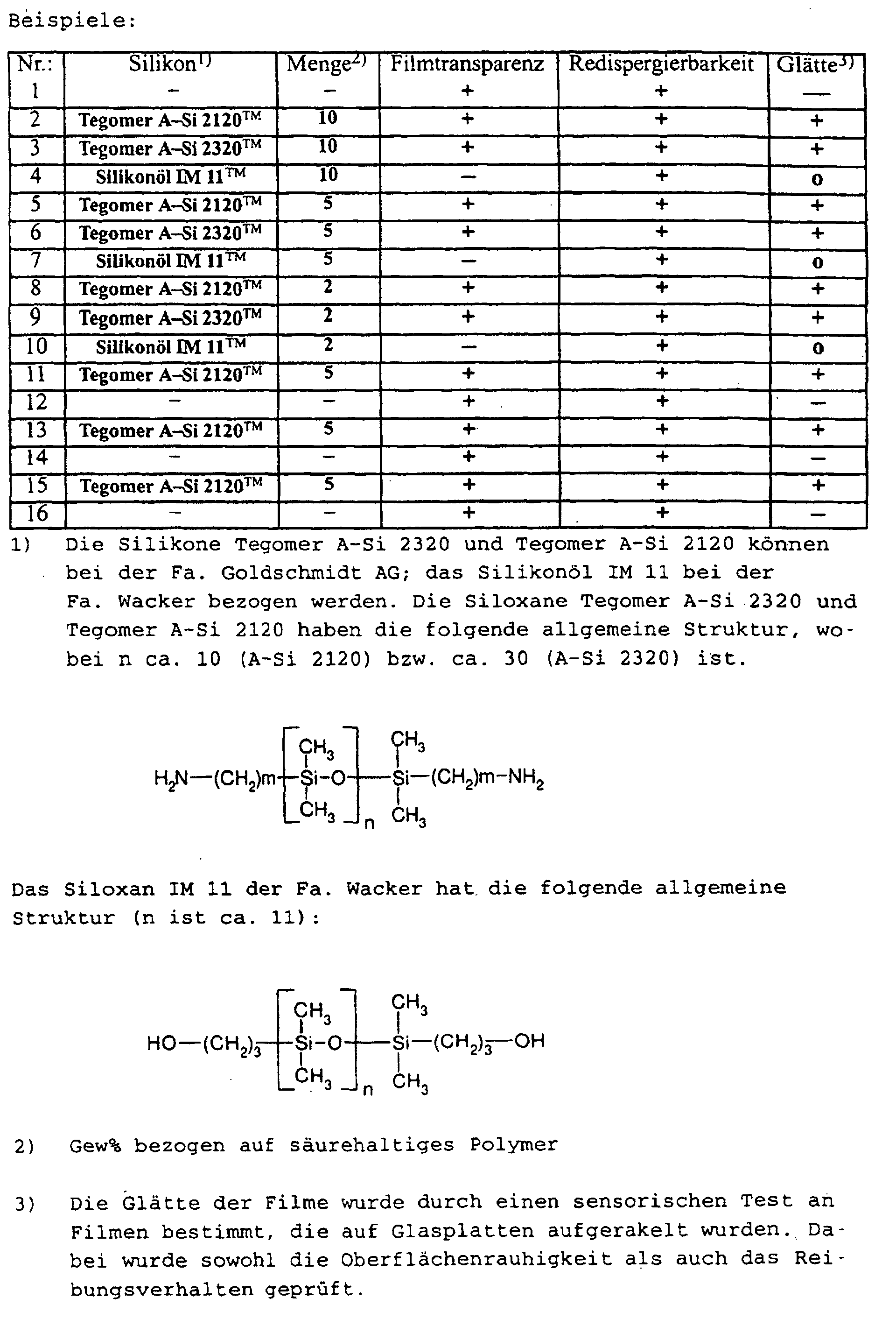

### Beispiel 1:

Eine 10 Gew.% ige Lösung eines t-Butylacrylat/Ethylacrylat/Methacrylsäure Terpolymers (Luvimer 100 P™ erhältlich von der BASF-AG, Ludwigshafen) in Ethanol wird mit 2-Amino-2-methyl-1-propanol (AMP) auf einen pH-Wert von 9,5 eingestellt. Diese Formulierung ergibt transparente, in Wasser redispergierbare Filme.

### Beispiele 2 - 4:

Analog zu Beispiel 1 wurden folgende Formulierungen mit 10 Gew.% der Siloxane Tegomer A-Si 2120, Tegomer A-Si 2320 und Silikon IM 11 hergestellt:
100 ml Ethanol
10 g eines t-Butylacrylat/Ethylacrylat/Methacrylsäure Terpolymers
   (Luvimer™ erhältlich von der BASF-AG, Ludwigshafen)
1 g des entsprechenden Siloxans
AMP ad pH 9,5

### Beispiele 5-7:

Analog zu Beispiel 1 wurden folgende Formulierungen mit 5 Gew.% der Siloxane Tegomer A-Si 2120, Tegomer A-Si 2320 und Silikon IM 11 hergestellt:
100 ml Ethanol
10 g eines t-Butylacrylat/Ethylacrylat/Methacrylsäure Terpolymers
   (Luvimer 100 P™ erhältlich von der BASF-AG, Ludwigshafen)
0,5 g Siloxan
AMP ad pH 9,5

### Beispiele 8-10:

Analog zu Beispiel 1 wurden folgende Formulierungen mit 2 Gew.% der Siloxane Tegomer A-Si 2120, Tegomer A-Si 2320 und Silikon IM 11 hergestellt:
100 ml Ethanol
10 g eines t-Butylacrylat/Ethylacrylat/Methacrylsäure Terpolymers
   (Luvimer 100 P™ erhältlich von der BASF-AG, Ludwigshafen)
0,2 g Siloxan
AMP ad pH 9,5

### Beispiel 11:

Formulierung aus:
100 ml Ethanol
10 g eines Octylacrylamid/Acrylat/Butylaminoethylmethacrylat
Copolymers (Amphomer™ erhältlich von der Firma National Starch)
0,5 g Tegomer A-Si 2120
AMP ad pH 7,0

### Beispiel 12:

Formulierung aus:
100 ml Ethanol
10 g eines Octylacrylamid/Acrylat/Butylaminoethylmethyacrylat
Copolymers (Amphomer™ erhältlich von der Firma National Starch)
AMP ad pH 7,0

### Beispiel 13, 14:

Formulierungen wie in Beispiel 11 und 12 mit einem Vinylacetat/Vinylpropionat/Crotonsäure Terpolymer (Luviset™ CAP erhältlich von der Firma BASF-AG).

### Beispiel 15,16:

Formulierungen wie im Beispiel 11 und 12 mit einem Acrylsäure/Ethylacrylat/N-t-Butylacrylamid Terpolymer (Ultrahold™ 8 erhältlich von der Firma BASF-AG).

## Patentansprüche

1. Haarfestigungsmittel enthaltend neben üblichen Hilfs- und Zusatzstoffen
(A) 0,5 bis 15 Gew.-% carboxylgruppenhaltige Polymere, die in neutralisierter Form wasserlöslich oder wasserdispergierbar sind, mit einer Glasübergangstemperatur über 0° C,
(B) 0,5 bis 30 Gew.-% bezogen auf das eingesetzte Polymer (A) primäre, sekundäre oder tertiäre Aminogruppen enthaltende. Polysiloxane, der folgenden Struktur 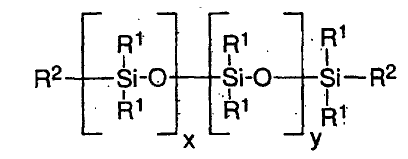 wobei die Reste
R¹ identisch oder unterschiedlich sein können und aus der Gruppe Methyl, Ethyl, Propyl, Butyl ausgewählt sind, R² Gruppen der allgemeinen Formel -(CH₂)ₙ-N(R⁴)₂ mit R⁴ gleich oder verschieden in der Bedeutung H oder aliphatische Kohlenwasserstoffreste mit 1-20 Kohlenstoffatomen,
n = 0-10 bedeuten und
x,y sind eine ganze Zahl, die so gewählt sind, das das Molekulargewicht des Polysiloxan-Blocks (B) zwischen 300 und 30000 liegt;
(C) 0 bis 50 % eines niedermolekularen Neutralisierungsmittels.

## Claims

1. A hairsetting composition comprising, in addition to customary auxiliaries and additives,
(A) from 0.5 to 15% by weight of carboxyl-containing polymers which are water-dispersible or water-soluble in neutralized form and have a glass transition temperature of above 0°C,
(B) from 0.5 to 30% by weight, based on the polymer (A) employed, of polysiloxanes containing primary, secondary or tertiary amino groups, of the following structure 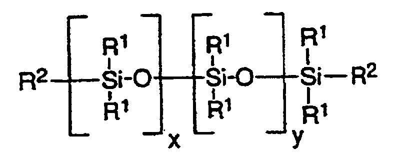 where the radicals
R¹ may be identical or different and are selected from the group consisting of methyl, ethyl, propyl and butyl,
R² are groups of the general formula -(CH₂)ₙ-N(R⁴)₂ where
R⁴ identically or differently are H or aliphatic hydrocarbon radicals having 1-20 carbon atoms,
n is 0-10 and
x, y are an integer selected such that the molecular weight of the polysiloxane block (B) is between 300 and 30 000;
(C) from 0 to 50% of a low molecular mass neutralizing agent.

## Revendications

1. Fixateur pour cheveux contenant, outre des adjuvants et additifs classiques,
(A) 0,5 à 15 % en poids de polymères contenant des groupes carboxyle, qui sont hydrosolubles ou hydrodispersables sous forme neutralisée, et ayant une température de transition vitreuse supérieure à 0°C,
(B) 0,5 à 30 % en poids par rapport au polymère (A) mis en oeuvre de polysiloxanes contenant des groupes amino primaires, secondaires ou tertiaires, ayant la structure suivante 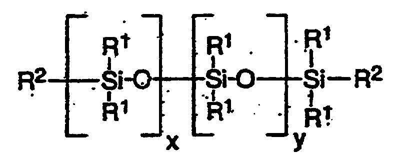 où les restes
R¹ peuvent être identiques ou différents et sont choisis dans le groupe méthyle, éthyle, propyle, butyle,
R² sont des groupes de formule générale -(CH₂)ₙ-N(R⁴)₂ avec R⁴ identiques ou différents et désignant H ou des restes hydrocarbonés aliphatiques ayant 1-20 atomes de carbone,
n = 0-10 et
x, y sont un nombre entier et sont choisis de telle manière que la masse moléculaire de la séquence polysiloxane (B) se situe entre 300 et 30000,
(C) 0 à 50 % d'un agent de neutralisation de faible masse moléculaire.
